# FASCICULE DE BREVET EUROPEEN

(11) **EP 2 385 094 B1**
(45) Date de publication et mention de la délivrance du brevet: **23.03.2016**
(21) Numéro de dépôt: 11290184.8
(22) Date de dépôt: 12.04.2011
(51) Int. Cl.: C10G 11/18, B01J 8/24, C07C 11/06, C07C 4/06

(54) **Procédé de craquage catalytique avec recycle d'une coupe oléfinique prélevée en amont de la section de séparation des gaz afin de maximiser la production de propylène**
Katkrackverfahren mit Recycling eines ölhaltigen Verschnittes, der vor dem Abschnitt der Gasabscheidung zur Maximierung der Propylenproduktion entnommen wird
Catalytic cracking method with recycling of an olefin cut taken upstream from the gas-separation section in order to maximise the production of propylene

(30) Priorité: 06.05.2010 FR 1001955
(43) Date de publication de la demande: 09.11.2011
(73) Titulaire: IFP Énergies nouvelles, 92852 Rueil-Malmaison Cedex (FR)
(72) Inventeur: Feugnet, Frédéric, 69004 Lyon (FR); Roux, Romain, 92500 Rueil Malmaison (FR)

(56) Documents cités:
- EP-A2- 0 704 517
- WO-A1-2006/104662
- FR-A1- 2 918 070
- FR-A1- 2 932 495
- US-A- 5 009 769

## Description

### DOMAINE DE L'INVENTION

La présente invention se situe dans le domaine du craquage catalytique de coupes pétrolières, plus particulièrement de coupes dites "lourdes", et dans le contexte de l'évolution du craquage catalytique vers la coproduction d'oléfines légères, notamment de propylène.

La charge principale d'une unité de FCC (abréviation de craquage catalytique en lit fluidisé) de coupes lourdes est généralement constituée d'un hydrocarbure ou d'un mélange d'hydrocarbures contenant essentiellement (c'est à dire au moins 80%) de molécules dont le point d'ébullition est supérieur à 340°C. Cette charge principale contient en outre des quantités de métaux (Ni+V) limitées, en concentration généralement inférieures à 50 ppm, préférentiellement inférieures à 20 ppm, et une teneur en hydrogène en général supérieure à 11 % poids, typiquement comprise entre 11,5% et 14,5%, et préférentiellement comprise entre 11,8% et 14% poids.

La teneur en carbone conradson (noté CCR en abrégé) de la charge (défini par la norme ASTM D 482) fournit une évaluation de la production de coke au cours du craquage catalytique. En fonction de la teneur en carbone conradson de la charge, le rendement en coke nécessite un dimensionnement spécifique de l'unité pour satisfaire le bilan thermique.

Ces coupes lourdes peuvent notamment provenir de la distillation atmosphérique, de la distillation sous vide, d'unité d'hydroconversion, d'unité de coking, d'unité d'hydrotraitement ou de désalphatage, mais avoir aussi une origine de type biomasse comme par exemple les huiles végétales ou la cellulose.

Les coupes lourdes constituant la charge principale d'une unité de craquage catalytique sont appelées dans la suite coupes lourdes conventionnelles et peuvent être traitées seules ou en mélange.

L'unité de craquage catalytique d'une raffinerie a pour objectif principal la production de bases pour essence, c'est à dire de coupes ayant un intervalle de distillation compris entre 35°C et 250°C.

De plus en plus, cet objectif premier s'accompagne d'un nouvel objectif qui est la co production d'oléfines légères, essentiellement de l'éthylène et du propylène.

La production d'essence est assurée par le craquage de la charge lourde dans le réacteur principal, appelé riser principal dans la suite du texte, en raison de la forme longiligne de ce réacteur et de son mode d'écoulement ascendant. Lorsque l'écoulement est descendant dans le réacteur principal on parle de "downer".

La coproduction de propylène est généralement assurée en rajoutant au système catalytique de base une zéolite à sélectivité de forme permettant d'améliorer la sélectivité en LPG ( abréviation de gaz de pétrole liquéfiés) et essence, ainsi qu'en sévérisant les conditions opératoires du riser principal, principalement par l'augmentation de la température en sortie dudit réacteur.

Afin d'atteindre des rendements en propylène plus importants, il est possible de recycler dans un réacteur additionnel, généralement un riser secondaire, une partie de la coupe essence produite par l'unité de craquage catalytique, ou une charge équivalente telle que des oligomères de C6, C7 et C8 provenant de la raffinerie.

La présente invention décrit un nouveau flux de recycle permettant de maximiser le rendement en propylène.

Il est connu de l'homme du métier que le recycle de l'essence catalytique du FCC dans la zone réactionnelle permet d'augmenter significativement le rendement en propylène en faisant appel à des conditions opératoires adaptées, c'est à dire une température plus élevée en sortie de riser et des rapports catalyseur/ charge (noté C/O) plus élevés.

L'intérêt de la présente invention est de prélever le nouveau recycle, non pas après la section de séparation, mais en amont de cette dernière, ce qui permet de s'affranchir des coûts de séparation et de bénéficier d'un recycle aux propriétés (composition chimique, notamment teneur en oléfines) aussi bonnes, voir meilleures (faible teneur en aromatiques) que celle du recycle d'essence selon l'art antérieur.

Le point de prélèvement du nouveau flux de recycle se situe au niveau de l'interétage du compresseur de gaz humide. Ce flux présente une composition riche en composés en C4 C5 C6 à C8 ayant une bonne oléfinicité (c'est à dire teneur en oléfines), et il est pratiquement exempt de composés aromatiques qui, après recycle, ont tendance à former principalement du coke et pénalisent ainsi le bilan thermique de l'unité.

### EXAMEN DE L'ART ANTERIEUR

Il est connu de l'homme du métier que le recycle de l'essence catalytique du FCC dans la zone réactionnelle permet d'augmenter significativement le rendement en propylène en faisant appel à des conditions opératoires adaptées, c'est à dire une température plus élevée en sortie de riser et des rapports catalyseur/ charge (C/O) plus élevés. Ce craquage d'essence de recycle peut être effectué dans le riser principal de l'unité ou dans un riser dédié.

L'art antérieur concernant les unités de craquage catalytique à deux risers, l'un conventionnel pour l'obtention d'essence, l'autre travaillant en conditions plus sévères pour l'obtention d'oléfines légères, est notamment décrit dans le brevet FR 2.918.070

On trouve dans ce texte les notions de riser principal travaillant sur une charge lourde, et de riser secondaire travaillant à haute sévérité sur une charge en partie constituée par le recycle de l'essence produite au riser principal.

Par ailleurs, l'optimisation de manière indépendante des conditions opératoires de deux risers fonctionnant en parallèle est décrite dans la demande de brevet FR 2.932.495.

La configuration downer est décrite dans les brevets EP 0 861 310 B1, US6, 664,171 B1. L'agencement d'un tube interne dans un riser est décrit dans le brevet US7, 008, 527 B2

### DESCRIPTION SOMMAIRE DE LA FIGURE

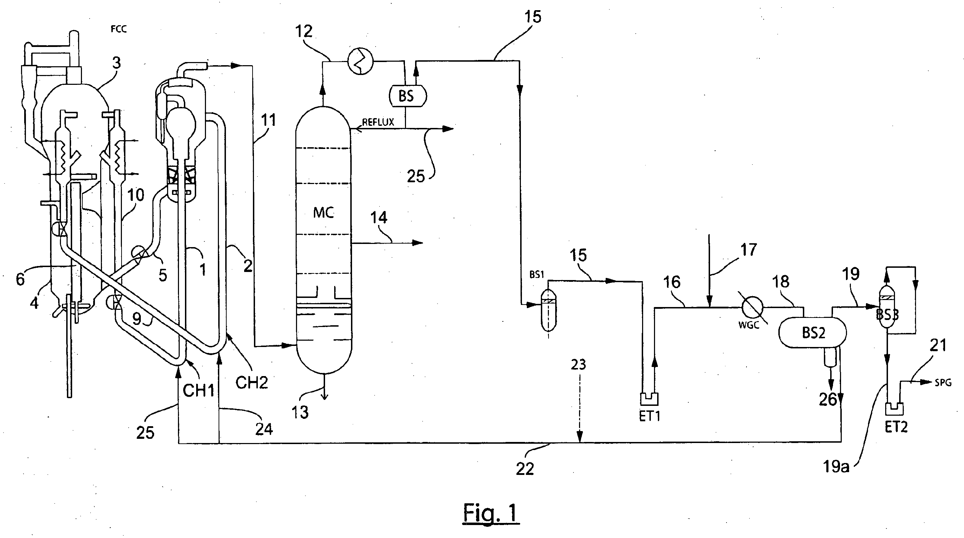

La figure 1 est un schéma du procédé selon l'invention dans lequel on montre le prélèvement de la coupe C4 C5 C6 oléfinique au niveau de l'interétage du compresseur de gaz humide et son recyclage dans le riser principal d'une unité de FCC.

### DESCRIPTTON SOMMAIRE DE L'INVENTION

La présente invention s'applique à des unités FCC fonctionnant avec un seul réacteur (en écoulement ascendant ou en écoulement descendant), et à des unités FCC fonctionnant avec deux réacteurs.

Dans la suite du texte, on parlera de réacteur principal noté (1) pour désigner le réacteur orienté vers la conversion de la charge principale, et de réacteur secondaire noté (2) pour désigner le réacteur dédié à la production de propylène en craquant une coupe de recycle. Généralement, lorsque les unités FCC fonctionnent avec deux réacteurs, un principal et un secondaire, ces réacteurs sont à écoulement ascendants, mais une unité qui ferait appel à deux réacteurs à écoulement descendant reste dans le cadre de la présente invention. Il est aussi possible de considérer le cas d'une unité avec un réacteur ascendant et un autre réacteur descendant.

Typiquement, le riser principal fonctionne avec un rapport catalyseur sur charge compris entre 4 et 15, et préférentiellement compris entre 5 et 10, et avec des températures de sortie riser (notée TS) comprises entre 510°C et 580°C, et préférentiellement comprises entre 520°C et 570°C.

Les conditions optimales de production de propylène dans le riser secondaire sont obtenues pour des températures de sortie dudit riser secondaire comprises entre 550°C et 650°C, et préférentiellement comprises entre 580°C et 610°C, des temps de contact compris entre 20 ms et 500 ms, préférentiellement compris entre 50 ms et 200 ms (ms = milliseconde), et des flux de solide compris entre 150 et 600 kg/s/m2.

Le temps de contact est défini comme le rapport du volume de catalyseur présent dans le réacteur sur le débit volumétrique de fluide traversant le réacteur aux conditions de mise en oeuvre de la réaction de craquage.

L'ensemble de ces conditions conduit à opérer le riser secondaire à des ratios catalyseur sur charge (noté C/O) compris entre 8 et 35, et préférentiellement compris entre 10 et 25.

Dans le cas d'un downer principal, il fonctionne avec un rapport catalyseur sur charge compris entre 5 et 40, et préférentiellement compris entre 10 et 30, et avec des températures de sortie downer (notée TS) comprises entre 500°C et 650°C, et préférentiellement comprises entre 550°C et 630°C.

Les conditions optimales de production de propylène dans un downer secondaire sont obtenues pour des températures de sortie dudit downer secondaire comprises entre 550°C et 650°C, et préférentiellement comprises entre 580°C et 630°C, des temps de contact compris entre 20 ms et 800 ms, préférentiellement compris entre 50 ms et 500 ms (ms = milliseconde). La présente invention consiste à recycler dans la section réactionnelle d'une unité FCC un flux de composés oléfiniques en C4, C5 et C6 principalement. Ce flux de recycle est prélevé au niveau de l'inter étage du compresseur de gaz humide (noté CGH).

L'intérêt de la présente invention est de prélever ce recycle, non pas après la section de séparation, mais en amont de cette dernière, ce qui permet de s'affranchir des coûts de séparation et de bénéficier d'un recycle aux propriétés (composition) aussi bonnes, voir meilleures (faible quantité d'aromatiques) que celles du recycle d'essence.

Par simplification on appelle dans la suite du texte ce flux, flux d'interétage.

Le flux provenant de l'inter étage du compresseur de gaz humide est largement composé d'oléfines en C4 et C5, typiquement dans une proportion allant de 30% à 80%.

Ces composés en C4 et C5 présentent une forte oléfinicité, c'est à dire une forte proportion de composés insaturés qui peut s'élever de 50% à 80 % poids pour la coupe en C4, et de 40% à 65 % poids pour la coupe en C5. Ces composés insaturés du flux d'interétage sont soumis dans des conditions opératoires particulières dans un riser de FCC à des réactions d'oligomérisation conduisant à la formation de composés à longueur de chaîne carbonée plus grande. Ces composés oligomérisés subissent à leur tour des réactions de craquage catalytique conduisant à la formation de quantités significatives de propylène.

Les molécules oléfiniques en C4, C5 et C6 contenues dans le flux d'interétage en question peuvent être recyclées soit dans le réacteur principal, soit dans le réacteur secondaire lorsque l'unité de FCC comprend déjà un tel réacteur secondaire.

Dans le cas d'un recyclage dans le réacteur principal, ce recycle pourra se faire soit directement en mélange avec la charge lourde, soit en amont des injecteurs de ladite charge lourde par l'intermédiaire d'injecteurs dédiés ou d'un tube interne au riser.

Dans le cas d'un recyclage dans le riser secondaire, il pourra s'agir soit d'un riser dédié, c'est-à-dire traitant uniquement le recycle en question, soit d'un riser secondaire convertissant déjà une charge légère telle que, par exemple, une partie de l'essence produite au riser principal, en vue de la production de propylène.

Afin de garantir un débit stable du flux d'interétage au riser principal ou secondaire (qui pourrait éventuellement varier en raison de fluctuations de fonctionnement de l'unité et du compresseur), il est possible d'associer au flux d'interétage un appoint qui peut venir de la partie avale du gaz plant, préférentiellement une essence catalytique. Cet appoint peut aussi être constitué de n'importe quel composé liquide pétrolier ou provenant de la biomasse avec de préférence une teneur en oléfines supérieure à 20% et un nombre d'atomes de carbone inférieur à 12, afin d'augmenter la potentiel de production de propylène.

Cet appoint peut être prélevé à la tête du splitter d'essence de FCC, au fond du dépropaniseur. Il peut également provenir d'une unité d'oligomérisation ou de Pygas ex Steam Cracker.

### DESCRIPTION DETAILLEE DE L'INVENTION

La description qui suit est faite au moyen de la figure 1.

La figure 1 représente une unité de craquage catalytique à deux risers (FCC), un riser principal (1) et un riser secondaire (2). Le riser principal (1) est alimenté en catalyseur régénéré à partir de la ligne (10), et le riser secondaire est alimenté en catalyseur régénéré à partir de la ligne (9). La zone de régénération possède deux étages, un premier étage (4) et un second étage (3) connecté au premier étage par une ligne de transfert du catalyseur (6).

Le catalyseur est transféré dans la zone de régénération en sortie de la zone de stripage (8) par la ligne de transfert (5).

Le riser principal (1) est alimenté par une charge conventionnelle (CH1) et le riser secondaire (2) est alimentée par une charge plus légère notée (CH2).

Le flux d'effluents (11) quittant l'unité de FCC est introduit dans la colonne de séparation MC de laquelle on extrait un flux de tête noté (12), un ou plusieurs flux intermédiaires (14) et un flux de fond (13). Les flux intermédiaires (14) et le flux de fond (13) ne seront pas décrits davantage dans la mesure où ils n'interviennent pas dans la présente invention.

On reprend donc la description au niveau du flux de tête (12) qui est condensé dans le ballon (BS) et séparé en deux phases.
- La phase liquide alimente pour une partie le reflux de la colonne (MC), et pour une autre partie (25) la séparation située en aval du compresseur de gaz humide (CGH) qui comporte deux étages (ET1, ET2).
- La phase gazeuse (15) issue du ballon (BS) est dirigée vers le premier étage du compresseur de gaz (ET1) après avoir été débarrassée des particules liquides qu'elle peut contenir dans le ballon séparateur (BS1).

Le flux compressé (16) issu du premier étage (ET1) est dirigé vers un second ballon séparateur (BS2) duquel on extrait 3 flux:
- un flux (19) qui est dirigé vers le second étage de compression (ET2) après être passé dans le ballon séparateur (BS3), devenant alors le flux 19a débarrassé des particules liquide. Le flux (19a) est compressé dans le deuxième étage de compression (ET2) pour délivrer le flux compressé (21) qui rejoint la section de récupération des gaz craqués (SRG),
- un flux (20) qui est dirigé vers la section de traitement des eaux usées (non représentée sur la figure 1),
- un flux (22) qui est recyclé vers le riser principal (1) ou le riser secondaire (2), ou partie vers le riser principal par le flux (25), et partie vers le riser secondaire par le flux (24) et qui constitue le flux d'interétage.

Dans la mesure où, comme on le voit clairement sur la figure 1, ce flux (22) est bien prélevé entre les deux étages de compression ET1 et ET2 du compresseur de gaz, il est nommé flux d'interétage.

L'invention peut donc se définir de manière générale comme un procédé de production d'essence et de coproduction de propylène faisant appel à une unité de craquage catalytique possédant au moins un réacteur principal fonctionnant à courant ascendant (appelé "riser") ou à courant descendant (appelé "downer") traitant une charge lourde conventionnelle, et dans laquelle le réacteur principal traite en outre, une charge constituée de molécules oléfiniques majoritairement en C4, C5 et C6 introduite en mélange avec la charge lourde ou en amont de ladite charge lourde, la dite charge oléfinique étant prélevée au niveau de l'interétage du compresseur de gaz humide (CGH) faisant partie de la section de traitement de gaz (SRG) associée à l'unité de FCC et constituant le flux d'interétage.

Dans une variante du procédé de production d'essence et de coproduction de propylène selon l'invention, la coupe oléfinique en C4, C5 et C6 est introduite en amont de la charge principale par l'intermédiaire d'un tube interne audit riser principal débouchant de 1m à 0,5 m avant le niveau des injecteurs de la charge principale.

De manière générale, si le réacteur principal fonctionne en courant descendant ("downer"), il fonctionne aux conditions opératoires suivantes: température de sortie du réacteur comprise entre 580°C et 630°C et rapport C/O compris entre 15 et 40, et préférentiellement compris entre 20 et 30, temps de résidence compris entre 0,1 et 1 s et préférentiellement compris entre 0,2 et 0,7 s.

Dans une variante du procédé de production d'essence et de coproduction de propylène selon l'invention, ledit procédé fait appel à une unité de craquage catalytique possédant un riser principal (1) traitant une charge conventionnelle (CH1) et un riser secondaire(2) fonctionnant en parallèle du riser principal (1) traitant une charge (CH2) plus légère que la charge lourde ( CH1) et travaillant à des conditions opératoires plus sévères que celles du riser principal, ledit riser secondaire (2) traitant la coupe oléfinique C4 C5 C6, représenté par le flux (22) en provenance de l'interétage du compresseur de gaz humide (CGH).

Toujours dans la variante où le procédé selon l'invention fait appel à une unité de craquage catalytique utilisant un riser principal (1) et un riser secondaire (2), le dit riser secondaire peut traiter en mélange la coupe oléfinique C4 C5 C6 (flux 22) en provenance de l'interétage du compresseur de gaz humide (CGH), une coupe essence et ou un oligomerat C5, C6 , C7 ou C8 recyclés non représenté sur la figure 1.

Toujours dans la variante où le procédé selon l'invention fait appel à une unité de craquage catalytique utilisant un riser principal (1) et un riser secondaire (2), il est possible d'ajouter au flux d'interétage un flux d'appoint (23) constitué d'essence de recycle de manière à garantir que la somme des deux flux, c'est à dire le flux d'interétage (22) plus le flux d'appoint (23), est constante à plus ou moins 10% près.

Toujours dans la variante où le procédé selon l'invention fait appel à une unité de craquage catalytique utilisant un riser principal (1) et un riser secondaire (2), le flux d'appoint (23) peut être constitué d'une coupe hydrocarbonée pétrolière ou d'origine biomasse, ayant une teneur en oléfines supérieure à 20% poids et contenant des composés à moins de 12 atomes de carbone.

Toujours dans la variante où le procédé selon l'invention fait appel à une unité de craquage catalytique utilisant un riser principal (1) et un riser secondaire (2), le flux d'appoint (23) peut être prélevé soit à la tête du splitter d'essence de FCC, soit au fond du dépropaniseur faisant partie de la section de traitement des gaz ("gaz plant").

Toujours dans la variante où le procédé selon l'invention fait appel à une unité de craquage catalytique utilisant un riser principal (1) et un riser secondaire (2), le flux d'appoint (23) peut provenir d'une unité d'oligomérisation ou de Pygas ex Steam Cracker.

Dans la variante où le procédé selon l'invention fait appel à une unité de craquage catalytique utilisant un riser principal (1) et un riser secondaire (2), le riser secondaire (2) fonctionne avec un temps de contact compris entre 20 et 500 ms, préférentiellement entre 50 ms et 200 ms, et des flux de solide comprises entre 150 et 600 kg/s. m2.

Dans la variante où le procédé selon l'invention fait appel à une unité de craquage catalytique utilisant un riser principal (1) et un riser secondaire (2), le rapport C/O du riser principal est compris entre 6 et 14, préférentiellement compris entre 7 et 12, et le rapport C/O du riser secondaire est compris entre 8 et 35, préférentiellement compris entre 10 et 25.

Dans la variante où le procédé selon l'invention fait appel à une unité de craquage catalytique utilisant un riser principal (1) et un riser secondaire (2), la température de sortie du riser principal est comprise entre 510°C et 580°C, préférentiellement comprise entre 520°C et 570°C, et la température de sortie du riser secondaire est comprise entre 550°C et 650°C, préférentiellement comprise entre 580 °C et 610°C.

### EXEMPLE

Pour illustrer la présente invention on a fait appel à 5 exemples, notés 1, 2, 3, 4 et 5.

### Exemple 1:

Ce premier exemple constitue le cas de base et correspond à une unité de FCC mono riser d'une capacité de 70000 BPSD, soit 500 m3/heure, (BPSD abréviation de barils par jour), traitant une charge résidu fonctionnant en conditions maxi propylène, c'est à dire avec un système catalytique contenant une zéolite à sélectivité de forme afin d'améliorer la sélectivité LPG sur essence et opérant dans des conditions opératoires plus sévères que les conditions standards de fonctionnement maxi essence.

Les caractéristiques principales de la charge ainsi que les conditions opératoires considérées sont présentées respectivement dans le Tableau 1 et le Tableau 2 ci dessous.

**Tableau 1 : Caractéristiques principales de la charge lourde**

| | | |
|---|---|---|
| Densité 15/4 °C | | 0,9305 |
| Soufre | %poids | 1,671 |
| Azote | ppm poids | 1222 |
| Carbone Conradson | %poids | 4,36 |
| Nickel | ppm | 12 |
| Vanadium | ppm | 19 |
| Viscosité (@ 50°C) | cSt | 84 |
| Hydrogène | %poids | 12,33 |

**Tableau 2 : Conditions opératoires Exemple1**

| | | **Exemple 1** |
|---|---|---|
| Capacité unité | Baril/j | 70000 |
| Débit de charge | t/h | 431,6 |
| Pression sortie riser | bar g | 1,4 |
| Température sortie riser | °C | 550 |
| Température de préchauffe de la charge | °C | 260 |
| Débit de vapeur (MP) | tonne/h | 29,6 |
| Ratio massique débit de catalyseur sur débit de charge | | 8 |

Dans ces conditions, les débits des produits sortie unité sont donnés dans le Tableau 3 ci dessous.

**Tableau 3 : Débit des produits de l'Exemple 1**

| **kg/h** | **Exemple 1 Cas de base** |
|---|---|
| **Gaz secs** | **22446** |
| NH3 | 43 |
| H2S | 3913 |
| H2 | 516 |
| C1 | 6020 |
| C2 | 4988 |
| C2= | 6966 |
| **GPL** | **133171** |
| C3 | 7568 |
| **C3=** | **46612** |
| C4 | 17243 |
| C4= | 61748 |
| **LIQUIDE** | **236543** |
| PI-160 | 113004 |
| 160-220 | 39302 |
| PI-220°C | 152306 |
| 220-360 | 54653 |
| 360 + | 29584 |
| Coke | 37840 |
| TOTAL | 430000 |

### Exemple 2:

L'exemple 2 correspond à l'exemple 1 avec un recyclage du flux d'interétage en amont de la charge principale.

Les conditions opératoires du riser restent identiques à celles de l'exemple 1.

Dans ces conditions, le débit du flux d'interétage correspond à 45 tonnes par heure et sa composition est fournie dans le Tableau 4 ci dessous.

**Tableau 4 : Composition du flux d'interétage Exemple 2**

| **Composition du flux d'interétage** | |
|---|---|
| % poids | |
| C3 totaux | 4 |
| C4 totaux | 20 |
| C5 totaux | 30 |
| C6-C8 | 46 |
| C3= dans les C3 totaux | 86 |
| C4= dans les C4 totaux | 60 |
| C5= dans les C5 totaux | 51 |
| Débit de recycle, t/h | 45 |

Les débits des produits sortie unité avec recycle sont donnés et comparés à ceux du cas de base dans le Tableau 5 ci dessous.

**Tableau 5 : Comparaison Exemple 1 et Exemple 2**

| kg/h | **Exemple1** | **Exemple 2** | **Delta** |
|---|---|---|---|
| | **Cas de base** | **Flux d'interétage coprocessé en amont de la charge lourde** | **%** |
| **Gaz secs** | **22446** | **23627** | **5.3** |
| NH3 | 43 | 43 | 0.0 |
| H2S | 3913 | 3913 | 0.0 |
| H2 | 516 | 571 | 10.6 |
| C1 | 6020 | 6511 | 8.2 |
| C2 | 4988 | 5243 | 5.1 |
| **C2=** | **6966** | **7346** | **5.5** |
| **GPL** | **133171** | **134432** | **0.9** |
| C3 | 7568 | 7927 | 4.7 |
| **C3=** | **46612** | **48616** | **4.3** |
| C4 | 17243 | 17855 | 3.5 |
| C4= | 61748 | 60035 | -2.8 |
| **LIQUIDE** | **236543** | **233089** | **-1.5** |
| PI-160 | 113004 | 107500 | -4.9 |
| 160-220 | 39302 | 40752 | 3.7 |
| PI-220°C | 152306 | 148252 | -2.7 |
| 220-360 | 54653 | 55110 | 0.8 |
| 360 + | 29584 | 29727 | 0.5 |
| Coke | 37840 | 38852 | 2.7 |
| TOTAL | 430000 | 430000 | 0.0 |

Dans ces conditions, la comparaison des tableaux 3 et 5 montre que le gain en propylène est de plus de 4 pourcents, ce qui est évidemment très significatif sur des débits industriels.

Une perte en C4 et C5 (au travers de la coupe PI-160°C) est bien évidemment observée puisque ces composés ont été recyclés et craqués.

Une augmentation des gaz secs et du coke est également observée, mais reste dans les limites acceptables de l'unité, notamment du point de vue du bilan thermique.

Le gain en éthylène qui est également un produit valorisable augmente très significativement avec une hausse de plus de 5%.

Le recycle du flux d'interétage (22) en amont de la charge principale (CH1) permet donc de remplir pleinement l'objectif recherché de maximiser la production de propylène tout en s'affranchissant des coûts de séparation par rapport à un recycle classique d'essence catalytique.

### Exemple 3:

L'exemple 3 est semblable à l'exemple 2 à la différence que cette fois le flux d'interétage est recyclé dans un riser secondaire (2) dédié et craqué dans des conditions opératoires optimisées, à savoir une température sortie riser de 590°C et un temps de contact de 250ms. Les débits de produits obtenus sont présentés et comparés à ceux de l'exemple 1 dans le Tableau 6.

**Tableau 6 : Comparaison Exemple 1 et Exemple 3**

| **kg/h** | **Exemple1** | **Exemple 3** | **Delta** |
|---|---|---|---|
| | **Cas de base** | **Flux d'interétage craqué dans un riser secondaire dédié** | **%** |
| **Gas secs** | **22446** | **24133** | **7,5** |
| NH3 | 43 | 43 | 0,0 |
| H2S | 3913 | 3913 | 0,0 |
| H2 | 516 | 594 | 15,2 |
| C1 | 6020 | 6721 | 11,7 |
| C2 | 4988 | 5352 | 7,3 |
| C2= | 6966 | 7509 | 7,8 |
| **GPL** | **133171** | **134973** | **1,4** |
| C3 | 7568 | 8080 | 6,8 |
| **C3=** | **46612** | **49475** | **6,1** |
| C4 | 17243 | 18117 | 5,1 |
| C4= | 61748 | 59301 | -4,0 |
| **LIQUIDE** | **236543** | **231609** | **-2,1** |
| PI-160 | 113004 | 105141 | -7,0 |
| 160-220 | 39302 | 41373 | 5,3 |
| PI-220°C | 152306 | 146514 | -3,8 |
| 220-360 | 54653 | 55306 | 1,2 |
| 360+ | 29584 | 29789 | 0,7 . |
| Coke | 37840 | 39286 | 3,8 |
| TOTAL | 430000 | 430000 | 0,0 |

Lorsque le flux d'interétage est recyclé dans un riser dédié (2) et soumis à des conditions opératoires optimisées, le gain en propylène par rapport au cas de base (Exemple1) est de plus de 6% . Il est donc amélioré par rapport au gain de 4% observé lorsque ce même recycle est craqué dans le riser principal en amont de la charge lourde.

Dans ces conditions, l'éthylène augmente également plus largement avec un gain de près de 8 % comparé aux 5% de l'exemple 2.

L'augmentation des gaz secs et du coke est plus importante que dans l'exemple 2 mais cette hausse reste limitée.

Le recycle de la coupe riche en C4 et C5 dans un riser dédié permet donc des gains en propylène plus important que lorsque cette charge est craqué dans un riser unique en amont de la charge principale.

### Exemple 4:

L'exemple suivant constitue un deuxième cas de base et correspond à une unité de FCC comportant deux risers, un riser principal (1) alimenté par une charge résidu (CH1), la même que pour l'exemple 1, et un riser secondaire (2) dans lequel une partie de l'essence catalytique produite (CH2) est recyclée et craquée dans des conditions sévères. Comme pour l'exemple 1, le système catalytique comprend une zéolite à sélectivité de forme afin d'optimiser le fonctionnement en maxi propylène de l'unité.

Les conditions opératoires de l'exemple 4 sont décrites dans le Tableau 7.

**Tableau 7 : Conditions opératoires de l'exemple 4**

| Cas | | **Exemple 4** |
|---|---|---|
| Capacité unité | Baril/j | 70 000 |
| | | |

| **Riser principal** | | |
|---|---|---|
| Débit de charge résidu | t/h | 431,6 |
| Pression sortie riser | bar g | 1,4 |
| Température sortie riser | °C | 550 |
| Température de préchauffe de la charge | °C | 260 |
| Débit de vapeur (MP) | t/h | 29,6 |
| Ratio massique débit de catalyseur sur débit de charge | | 8 |
| | | |

| **Riser secondaire** | | |
|---|---|---|
| Débit de recycle d'essence catalytique | t/h | 108 |
| Température sortie riser | °C | 590 |
| Température du recycle | °C | 53 |
| Débit de vapeur (MP) | t/h | 3,3 |
| Ratio massique débit de catalyseur sur débit de charge | | 12 |

Sur cette base, le débit des produits de sortie unité sont donnés dans le Tableau 8

**Tableau 8 : Débit des produits de l'Exemple 4**

| **kg/h** | **Exemple 4** |
|---|---|
| | **Cas de base dual riser recycle essence** |
| **Gaz secs** | **24940** |
| NH3 | 43 |
| H2S | 3913 |
| H2 | 602 |
| C1 | 7095 |
| C2 | 5504 |
| C2= | 7783 |
| **GPL** | **142115** |
| C3 | 8600 |
| **C3=** | **51600** |
| C4 | 18060 |
| C4= | 63855 |
| **LIQUIDE** | **224632** |
| PI-160 | 97782 |
| 160-220 | 41667 |
| PI-220°C | 139449 |
| 220-360 | 55556 |
| 360 + | 29627 |
| Coke | 38313 |
| TOTAL | 430000 |

### Exemple 5:

L'exemple 5 reprend le cas de l'exemple 4 mais cette fois avec un recycle du flux d'interétage dans le riser secondaire en mélange avec de l'essence issue de l'unité de FCC. Par rapport à l'exemple 2, ce recycle a changé en composition mais le même débit de recycle a été considéré.

La composition de ce recycle est fournie dans le Tableau 9.

**Tableau 9 : Composition du flux d'interétage Exemple 5**

| **Composition du flux d'interétage** | |
|---|---|
| wt% | |
| C3 totaux | 6 |
| C4 totaux | 20 |
| C5 totaux | 56 |
| C6-C8 | 18 |
| C3= dans les C3 totaux | 84 |
| C4= dans les C4 totaux | 80 |
| C5= dans les C5 totaux | 51 |
| Débit du recycle t/h | 45 |

Les débits des produits sortie unité avec recycle sont donnés et comparés à ceux de l'exemple 4 dans le Tableau 10

**Tableau 10 : Comparaison Exemple 4 et Exemple 5**

| **kg/h** | **Exemple 4** | **Exemple 5** | **Delta** |
|---|---|---|---|
| | **Cas de base dual riser recycle essence** | **Dual riser recycle d'essence ET flux d'interétage au riser secondaire** | |
| **Gaz secs** | **24940** | **26911** | **7,9** |
| NH3 | 43 | 43 | **0,0** |
| H2S | 3913 | 3913 | **0,0** |
| H2 | 602 | 713 | **18,4** |
| C1 | 7095 | 7921 | **11,6** |
| C2 | 5504 | 5995 | **8,9** |
| C2= | 7783 | 8326 | **7,0** |
| **GPL** | **142115** | **142487** | **0,3** |
| C3 | 8600 | 9119 | **6,0** |
| **C3=** | **51600** | **54550** | **5,7** |
| C4 | 18060 | 18828 | **4,3** |
| C4= | 63855 | 59989 | **-6,1** |
| **LIQUIDE** | **224632** | **220888** | **-1,7** |
| PI-160 | 97782 | 92349 | **-5,6** |
| 160-220 | 41667 | 42873 | **2,9** |
| PI-220°C | 139449 | 135222 | **-3,0** |
| 220-360 | 55556 | 55959 | **0,7** |
| 360+ | 29627 | 29707 | **0,3** |
| **Coke** | **38313** | **39714** | **3,7** |
| TOTAL | 430000 | 430000 | **0,0** |

Le recycle du flux d'interétage en mélange avec l'essence issue de l'unité de FCC permet une augmentation très nette du rendement en propylène avec une hausse de près de 6%. L'éthylène progresse de manière significative avec un gain de 7%. Les rendements en coke et gaz secs augmentent mais restent dans des limites acceptables. L'éthylène progresse de 7%, les gaz secs de près de 8% et le coke de moins de 4%. L'ensemble de ces exemples met en évidence que dans tous les cas de figures, le recyclage du flux d'interétage (22) soit au riser principal (1), soit au riser secondaire (2), soit pour partie au riser principal (1), et pour partie au riser secondaire (2), permet d'augmenter très significativement le rendement en propylène.

## Revendications

1. Procédé de production d'essence et de coproduction de propylène faisant appel à une unité de craquage catalytique possédant au moins un réacteur principal (1) fonctionnant en écoulement ascendant ("riser") ou descendant ("downer") et traitant une charge lourde conventionnelle (CH1), et éventuellement un riser secondaire (2) fonctionnant dans des conditions plus sévères que le riser principal (1) et traitant une charge plus légère (CH2), procédé dans lequel on traite outre la charge principale (CH1) et l'éventuelle charge plus légère (CH2), une charge constituée de molécules oléfiniques majoritairement en C4, C5 et C6, la dite charge oléfinique étant prélevée au niveau de l'interétage du compresseur de gaz humide faisant partie de la section de traitement de gaz (SRG) associé à l'unité de FCC et constituant le flux d'interétage (22).

2. Procédé de production d'essence et de coproduction de propylène selon la revendication 1 dans lequel lorsque l'unité de craquage catalytique possède uniquement un réacteur principal (1), la coupe oléfinique en C4, C5 et C6, correspondant au flux d'interétage (22), est introduite en mélange avec la charge lourde (CH1).

3. Procédé de production d'essence et de coproduction de propylène selon la revendication 1 dans lequel lorsque l'unité de craquage catalytique possède uniquement un réacteur principal (1), la coupe oléfinique en C4, C5 et C6, correspondant au flux d'interétage (22), est introduite en amont de la charge principale (CH1) par l'intermédiaire d'un tube interne audit riser principal (1) débouchant de 1m à 0,5 m avant le niveau des injecteurs de la charge principale (CH1).

4. Procédé de production d'essence et de coproduction de propylène selon la revendication 1, dans lequel, lorsque l'unité de craquage catalytique possède en outre un riser secondaire (2) traitant une charge plus légère (CH2), et travaillant à des conditions opératoires plus sévères que celles du riser principal, la coupe oléfinique en C4, C5 et C6, correspondant au flux d'interétage (22), est introduite dans le riser secondaire (2).

5. Procédé de production d'essence et de coproduction de propylène faisant appel à une unité de craquage catalytique possédant un riser principal (1) et un riser secondaire (2) selon la revendication 4, le dit riser secondaire (2) traitant en mélange la coupe oléfinique C4 C5 C6 correspondant au flux d'interétage 22), et une coupe essence et ou un oligomerat C5, C6 , C7 ou C8 recyclés.

6. Procédé de production d'essence et de coproduction de propylène faisant appel à une unité de craquage catalytique selon la revendication 1, dans lequel est ajouté au flux d'interétage (22) un flux d'appoint (23) constitué d'essence de recycle de manière à garantir que la somme des deux flux, c'est à dire le flux d'interétage (22) plus le flux d'appoint (23), est constante à plus ou moins 10% près.

7. Procédé de production d'essence et de coproduction de propylène faisant appel à une unité de craquage catalytique selon la revendication 6, dans lequel le flux d'appoint (23) est constitué d'une coupe hydrocarbonée pétrolière ou d'origine biomasse, ayant une teneur en oléfines supérieure à 20% poids et contenant des composés à moins de 12 atomes de carbone.

8. Procédé de production d'essence et de coproduction de propylène faisant appel à une unité de craquage catalytique selon la revendication 6, dans lequel le flux d'appoint (23) est prélevé soit à la tête du splitter d'essence de FCC, soit au fond du dépropaniseur faisant partie de la section de traitement des gaz (SRG).

9. Procédé de production d'essence et de coproduction de propylène faisant appel à une unité de craquage catalytique selon la revendication 6, dans lequel le flux d'appoint (23) provient d'une unité d'oligomérisation ou de Pygas ex Steam Cracker.

10. Procédé de production d'essence et de coproduction de propylène faisant appel à l'unité de craquage catalytique selon la revendication 4, dans laquelle le riser secondaire (2) fonctionne avec un temps de contact compris entre 20 et 500 ms, préférentiellement entre 50 ms et 200 ms, et des flux de solide comprises entre 150 et 600 kg/s. m2.

11. Procédé de production d'essence et de coproduction de propylène utilisant l'unité de craquage catalytique selon la revendication 4, dans lequel le rapport C/O du riser principal (1) est compris entre 6 et 14, préférentiellement compris entre 7 et 12, et le rapport C/O du riser secondaire (2) est compris entre 8 et 35, préférentiellement compris entre 10 et 25.

12. Procédé de production d'essence et de coproduction de propylène faisant appel à l'unité de craquage catalytique selon la revendication 4, dans lequel la température de sortie du riser principal (1) est comprise entre 510°C et 580°C, préférentiellement comprise entre 520°C et 570°C, et la température de sortie du riser secondaire (2) est comprise entre 550°C et 650°C, préférentiellement comprise entre 580 °C et 610°C.

## Patentansprüche

1. Verfahren zur Herstellung von Benzin und zur gleichzeitigen Herstellung von Propylen unter Verwendung einer Einheit zum katalytischen Cracken, die mindestens einen Hauptreaktor (1), der mit einer Aufstromsäule ("Riser") oder einer Abstromsäule ("Downer") arbeitet, bei der ein herkömmliches schweres Einsatzgut (CH1) behandelt wird, und gegebenenfalls einen sekundären Riser (2) aufweist, der unter strikteren Bedingungen arbeitet als der Haupt-Riser (1) und ein leichteres Einsatzgut (CH2) behandelt, ein Verfahren, bei dem neben dem Haupteinsatzgut (CH1) und dem gegebenenfalls vorhandenen leichteren Einsatzgut (CH2) auch ein Einsatzgut behandelt wird, das überwiegend aus C4-, C5- und C6-Olefinmolekülen besteht, wobei dieses olefinische Einsatzgut an der Zwischenstufe des Nassgasverdichters entnommen wird, der Teil das Gasbehandlungsabschnitts (SRG) ist, der mit der FCC-Einheit assoziiert ist und den Zwischenstufenstrom (22) bildet.

2. Verfahren zur Herstellung von Benzin und zur gleichzeitigen Herstellung von Propylen nach Anspruch 1, bei dem, wenn die Einheit zum katalytischen Cracken nur einen Hauptreaktor (1) aufweist, der C4-, C5-und C6-Olefinschnitt, der dem Zwischenstufenstrom (22) entspricht, gemischt mit dem schweren Einsatzgut (CH1) eingeführt wird.

3. Verfahren zur Herstellung von Benzin und zur gleichzeitigen Herstellung von Propylen nach Anspruch 1, bei dem, wenn die Einheit zum katalytischen Cracken nur einen Hauptreaktor (1) aufweist, der C4-, C5-und C6-Olefinschnitt, der dem Zwischenstufenstrom (22) entspricht, stromaufwärts des Haupteinsatzgutes (CH1) mithilfe eines Rohrs innerhalb des Haupt-Risers (1) eingeführt wird, das 1 m bis 0,5 m vor der Höhe der Injektoren des Haupteinsatzgutes (CH1) mündet.

4. Verfahren zur Herstellung von Benzin und zur gleichzeitigen Herstellung von Propylen nach Anspruch 1, wobei, wenn die Einheit zum katalytischen Cracken außerdem einen sekundären Riser (2) besitzt, der ein leichteres Einsatzgut (CH2) behandelt und bei strikteren Arbeitsbedingungen arbeitet als denen des Haupt-Risers, wird der C4-, C5- und C6-Olefinschnitt,der dem Zwischenstufenstrom (22) entspricht, in den sekundären Riser (2) eingeführt.

5. Verfahren zur Herstellung von Benzin und zur gleichzeitigen Herstellung von Propylen unter Verwendung einer Einheit zum katalytischen Cracken, die einen Haupt-Riser (1) und einen sekundären Riser (2) nach Anspruch 4 besitzt, wobei der sekundäre Riser (2) ein Gemisch aus dem C4-, C5-, C6-Olefinschnitt, der dem Zwischenstufenstrom (22) entspricht, und einem Benzinschnitt und/oder einem recycelten C5-, C6-, C7- oder C8-Oligomerat behandelt.

6. Verfahren zur Herstellung von Benzin und zur gleichzeitigen Herstellung von Propylen unter Verwendung einer Einheit zum katalytischen Cracken nach Anspruch 1, wobei zum Zwischenstufenstrom (22) ein Zusatzstrom (23) gegeben wird, der aus Recyclingbenzin besteht, um zu garantieren, dass die Summe der beiden Ströme, das heißt Zwischenstufenstrom (22) plus Zusatzstrom (23), konstant bei ungefähr 10 % liegt.

7. Verfahren zur Herstellung von Benzin und zur gleichzeitigen Herstellung von Propylen unter Verwendung einer Einheit zum katalytischen Cracken nach Anspruch 6, wobei der Zusatzstrom (23) aus einem Kohlenwasserstoffschnitt aus Erdöl oder aus Biomasse besteht, der einen Olefingehalt von mehr als 20 Gew.-% aufweist und der Verbindungen mit weniger als 12 Kohlenstoffatomen enthält.

8. Verfahren zur Herstellung von Benzin und zur gleichzeitigen Herstellung von Propylen unter Verwendung einer Einheit zum katalytischen Cracken nach Anspruch 6, wobei der Zusatzstrom (23) entweder am Kopf des Benzinsplitters der FCC oder am Boden des Entpropanisierers, der Teil des Gasbehandlungsabschnitts (SRG) ist, entnommen wird.

9. Verfahren zur Herstellung von Benzin und zur gleichzeitigen Herstellung von Propylen unter Verwendung einer Einheit zum katalytischen Cracken nach Anspruch 6, wobei der Zusatzstrom (23) aus einer Oligomerisierungseinheit oder einer Pygas-Einheit, z. B. einem Steam Cracker stammt.

10. Verfahren zur Herstellung von Benzin und zur gleichzeitigen Herstellung von Propylen unter Verwendung einer Einheit zum katalytischen Cracken nach Anspruch 4, wobei der sekundäre Riser (2) mit einer Kontaktzeit im Bereich zwischen 20 und 500 ms, vorzugsweise zwischen 50 ms und 200 ms arbeitet und der Feststoffstrom im Bereich zwischen 150 und 600 kg/s. m² liegt.

11. Verfahren zur Herstellung von Benzin und zur gleichzeitigen Herstellung von Propylen unter Verwendung einer Einheit zum katalytischen Cracken nach Anspruch 4, wobei das C/O-Verhältnis des Haupt-Risers (1) im Bereich zwischen 6 und 14, vorzugsweise im Bereich zwischen 7 und 12 liegt, und das C/O-Verhältnis des sekundären Risers (2) im Bereich zwischen 8 und 35, vorzugsweise im Bereich zwischen 10 und 25 liegt.

12. Verfahren zur Herstellung von Benzin und zur gleichzeitigen Herstellung von Propylen unter Verwendung einer Einheit zum katalytischen Cracken nach Anspruch 4, wobei die Auslasstemperatur des Haupt-Risers (1) im Bereich zwischen 510 °C und 580 °C, vorzugsweise im Bereich zwischen 520 °C und 570 °C liegt, und die Auslasstemperatur des sekundären Risers (2) im Bereich zwischen 550 °C und 650°C, vorzugsweise im Bereich zwischen 580 °C und 610 °C liegt.

## Claims

1. A process for the production of gasoline and the co-production of propylene employing a catalytic cracking unit having at least one principal reactor (1) functioning in upflow ("riser") mode or downflow ("downer") mode and treating a conventional heavy feed (CH1), and optionally a secondary riser (2) functioning under more severe conditions than the principal riser (1) and treating a lighter feed (CH2), in which process, in addition to the principal feed (CH1) and the optional lighter feed (CH2), a feed constituted mainly by C4, C5 and C6 olefinic molecules is treated, said olefinic feed being removed from the inter-stage of the wet gas compressor forming part of the gas treatment section (SPG) associated with the FCC unit and constituting the inter-stage stream (22).

2. A process for the production of gasoline and the co-production of propylene according to claim 1 in which, when the catalytic cracking unit has only one principal reactor (1), the olefinic C4, C5 and C6 feed corresponding to the inter-stage stream (22) is introduced as a mixture with the heavy feed (CH1).

3. A process for the production of gasoline and the co-production of propylene according to claim 1 in which, when the catalytic cracking unit has only one principal reactor (1), the olefinic C4, C5 and C6 feed corresponding to the inter-stage stream (22) is introduced upstream of the principal feed (CH1) via a pipe inside said principal riser (1) opening 1 m to 0.5 m before the level of the injectors for the principal feed (CH1).

4. A process for the production of gasoline and the co-production of propylene according to claim 1 in which, when the catalytic cracking unit further has a secondary riser (2) treating a lighter feed (CH2), and working under more severe operating conditions than those of the principal riser, the olefinic C4, C5 and C6 cut corresponding to the inter-stage stream (22) is introduced into the secondary riser (2).

5. A process for the production of gasoline and the co-production of propylene employing a catalytic cracking unit having a principal riser (1) and a secondary riser (2) according to claim 4, said secondary riser (2) treating the olefinic C4 C5 C6 cut corresponding to the inter-stage stream (22) and a recycled gasoline cut and/or a C5, C6, C7 or C8 oligomerizate cut as a mixture.

6. A process for the production of gasoline and the co-production of propylene employing a catalytic cracking unit according to claim 1, in which a makeup stream (23) constituted by recycled gasoline is added to the inter-stage stream (22) in order to ensure that the sum of the two streams, i.e. the inter-stage stream (22) plus the makeup stream (23), is constant to plus or minus 10%.

7. A process for the production of gasoline and the co-production of propylene employing a catalytic cracking unit according to claim 6, in which the makeup stream (23) is constituted by a hydrocarbon cut of oil or biomass origin having an olefins content of more than 20% by weight and containing compounds having less than 12 carbon atoms.

8. A process for the production of gasoline and the co-production of propylene employing a catalytic cracking unit according to claim 6, in which the makeup stream (23) is removed either from the head of the FCC gasoline splitter or from the bottom of the depropanizer forming part of the gas treatment section (SPG).

9. A process for the production of gasoline and the co-production of propylene employing a catalytic cracking unit according to claim 6, in which the makeup stream (23) derives from an oligomerization unit or from pygas, from a steam cracker.

10. A process for the production of gasoline and the co-production of propylene employing a catalytic cracking unit according to claim 4, in which the secondary riser (2) functions with a contact time in the range 20 to 500 ms, preferably in the range 50 ms to 200 ms, and with solid flow rates in the range 150 to 600 kg/s.m².

11. A process for the production of gasoline and the co-production of propylene employing a catalytic cracking unit according to claim 4, in which the C/O ratio of the principal riser (1) is in the range 6 to 14, preferably in the range 7 to 12, and the C/O ratio of the secondary riser (2) is in the range 8 to 35, preferably in the range 10 to 25.

12. A process for the production of gasoline and the co-production of propylene employing a catalytic cracking unit according to claim 4, in which the outlet temperature from the principal riser (1) is in the range 510°C to 580°C, preferably in the range 520°C to 570°C, and the outlet temperature from the secondary riser (2) is in the range 550°C to 650°C, preferably in the range 580°C to 610°C.
